(19) Europäisches Patentamt
European Patent Office
Office européen des brevets

(11) Veröffentlichungsnummer: **0 002 444**
**B1**

(12) # EUROPÄISCHE PATENTSCHRIFT

(45) Veröffentlichungstag der Patentschrift: 20.01.82

(21) Anmeldenummer: 78101270.3

(22) Anmeldetag: 02.11.78

(51) Int. Cl.³: C 07 D 233/74, C 07 D 233/76, C 07 D 233/78, C 08 G 73/06

(54) **Verfahren zur Herstellung von Hydantoinen und nach diesem Verfahren erhältliche Verbindungen.**

(30) Priorität: 12.11.77 DE 2750771

(43) Veröffentlichungstag der Anmeldung: 27.06.79 Patentblatt 79/13

(45) Bekanntmachung des Hinweises auf die Patenterteilung: 20.01.82 Patentblatt 82/3

(84) Benannte Vertragsstaaten: DE FR GB

(56) Entgegenhaltungen:
DE - A - 1 720 624
DE - A - 1 906 492
DE - A - 2 014 859
DE - A - 2 358 504
DE - A - 2 404 741
FR - A - 2 225 426
FR - A - 2 322 865

(73) Patentinhaber: **BAYER AG**
**Zentralbereich Patente, Marken und Lizenzen**
**D-5090 Leverkusen 1, Bayerwerk (DE)**

(72) Erfinder: **Lewalter, Jürgen, Dr.**
**Bergstrasse 88**
**D-5068 Odenthal (DE)**
Erfinder: **Merten, Rudolf, Dr.**
**Berta-von-Suttner-Strasse 55**
**D-5090 Leverkusen 1 (DE)**
Erfinder: **Zecher, Wilfried, Dr.**
**Treptower Strasse 6**
**D-5090 Leverkusen 1 (DE)**
Erfinder: **Dünwald, Willi, Dr.**
**Geschwister-Scholl-Strasse 16**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 444 B1

Verfahren zur Herstellung von Hydantoinen und nach diesem Verfahren erhältliche Verbindungen

Die Erfindung betrifft Kondensate mit mindestens einem Hydantoin- der Thiohydantoinring im Molekül, deren Herstellung aus ungesättigten Carbonsäuren und organischen Isocyanaten sowie deren Verwendung als biochemische Wirkstoffe bzw. zur Herstellung hitzeresistenter Beschichtungsmittel.

Verfahren zur Herstellung von Hydantoinen (J. Am. Chem. 45/383) bzw. Polyhydantoinen (BE 678 282) sind bekannt. Niedermolekulare Hydantoine werden bevorzugt im Pharma- und Pflanzenschutzbereich, höhermolekulare Hydantoine beispielsweise im wärmebeständigan Beschichtungsmittelsektor verwendet (franz. Pat. 1 484 694).

Überraschenderweise wurde gefunden, daß durch Umsetzung von ungesättigten Carbonsäure- bzw. Derivaten der allg. Formeln Ia und Ib

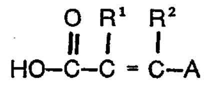 (Ia) oder (Ib) 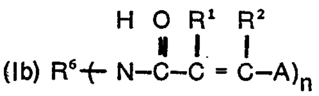 ,

mit organischen Isocyanaten der allg. Formel (V)

$$R^5(—NCO)_n \qquad (V)$$

bzw. Isocyanat-Abspaltern, in denen

$R_1$ und $R_2$ gleich oder verschieden Wasserstoff, Halogen, einen gegebenenfalls substituierten aliphatischen, aliphatisch-aromatischen, aromatischen oder heterocyclischen Rest und

A eine —CN—Gruppe, eine —COR—Gruppe mit R = Wasserstoff, ein aliphatischer, aliphatisch-aromatischer, aromatischer oder heterocyclischer Rest; oder einen gegebenenfalls substituierten aromatischen, aliphatischen, aliphatisch-aromatischen oder heterocyclischen Rest,

$R^5$ und $R^6$ für einen gegebenenfalls substituierten aliphatischen, aliphatisch-aromatischen, aromatischen oder heterocyclischen Rest,

m 1 oder 2 und

n eine ganze Zahl von 1—4 bedeuten,

gegebenenfalls in Anwesenheit eines Katalysators und ein oder mehreren Lösungsmitteln bei Temperatur von −20 bis 500°C Verbindungen erhalten werden, die aus 1—1000 wiederkehrenden Struktureinheiten (II)

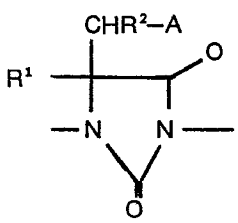 (IIa) bzw. 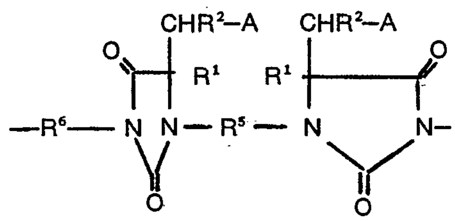 (IIb),

in denen

$R^1$, $R^2$, $R^5$, $R^6$ und A die oben angegebene Bedeutung haben, bestehen und die ggf. über wenigstens einen $R^5$— oder $R^6$—Rest verknüpft sind und ggf. endständige NCO—Gruppen aufweisen.

Die erfindungsgemäße Verfahrenstechnik kann formal durch die folgenden Gleichungen wiedergegeben werden. Die allgemeinen Reste haben die unter Formel (IIa/b) aufgeführte Bedeutung:

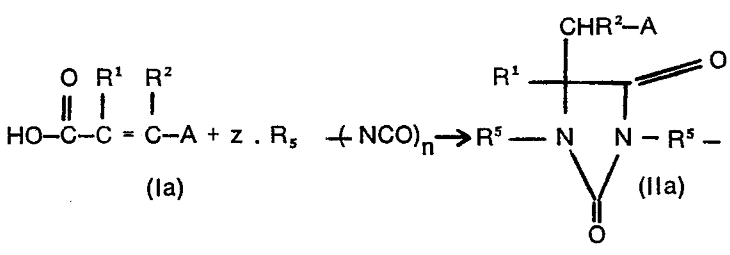

(Ia) (IIa)

bzw

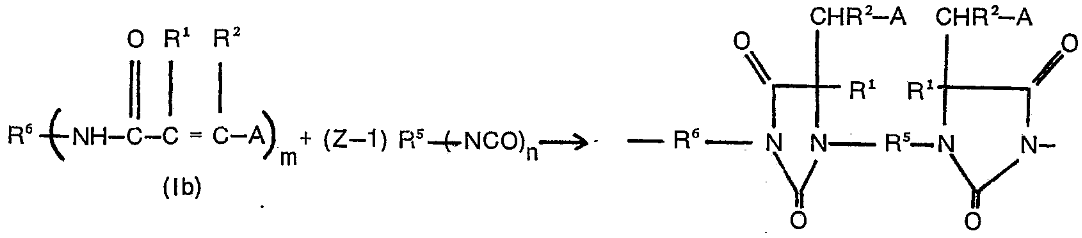

(Ib)

(IIb)

wobei
z für eine ganze Zahl von 1 bis 2 steht, für
n = 1 und m = 1 monomolekulare Verbindungen, und für
n > 1 höhermolekulare Verbindungen entstehen können, deren Hydantoinringe nur über $R^5$ oder über $R^5$ bzw. $R^6$-Reste verknüpft sind und statistische Endgruppen aufweisen. Dabei können die Hydantoinringe 1-, 3- sowie 3-, 1- verknüpft sein bzw. über eine statistische Folge aller Verknüpfungsmöglichkeiten.

Ein weiterer Gegenstand der Erfindung sind daher auch monomolekulare Hydantoine der allgemeinen Formel (III)

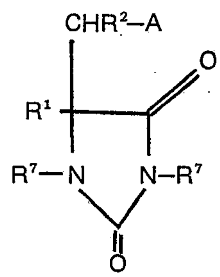

(III)

höhermolekulare Hydantoine mit den allgemeinen Formeln (IIa und IIb) sowie Polyisocyanate der allgemeinen Formeln (IVa und IVb)

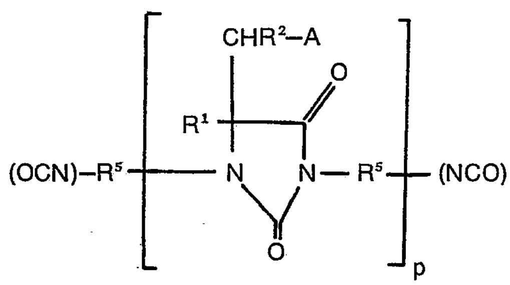

(IVa)

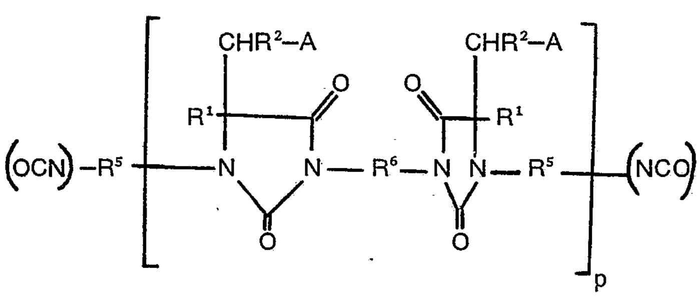

(IVb)

wobei A, $R^1$, $R^2$, $R^5$ und $R^6$ die oben angegebene Bedeutung hat und $R^7$ für $R^6$ und/oder $R^5$ und p für eine ganze Zahl von 1—100 stehen.

Die erfindungsgemäßen Hydantoine können eindeutig über die charakteristischen IR-Banden der Hydantoine identifiziert werden. Die höhermolekularen Hydantoine besitzen in 30 Gew.-%igen Lösungen mit Acetophenon, Butyrolacton, Caprolacton oder Benzoesäuremethylester bei 20°C Lösungsviskositäten von 200 bis 200 000 mPa s, vorzugsweise von 500 bis 50 000 mPa s.

Im einzelnen sind die für Y = OH erfindungsgemäß als Ausgangsmaterialien verwendeten ungesättigten Carbonsäuren, Verbindungen der allgemeinen Formel (Ia)

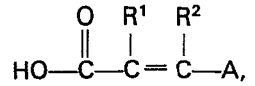

(Ia)

in der vorzugsweise

$R^1$ und $R^2$ gleich oder verschieden, Wasserstoff, Halogen, einen aliphatischen Rest mit $C_1$—$C_{20}$, aliphatisch-aromatischen Rest mit $C_7$—$C_{20}$, aromatischen Rest mit $C_6$—$C_{20}$ oder heterocyclischen Rest mit 4 bis 16 Ringgliedern und wenigstens einem N, O oder S-Atom und

A einen aliphatischen Rest mit $C_1$—$C_{20}$, einen aliphatisch-aromatischen Rest mit $C_7$—$C_{20}$, einen aromatischen Rest mit $C_6$—$C_{20}$, oder heterocyclischen Rest mit 4—16 Ringgliedern, die gegebenenfalls durch Halogen (Chlor, Brom), —$NO_2$, —$COOR_4$, —CN, —CO, —OH Gruppen mit $R_4 = R_1$ außer Halogen, substituiert sein können; eine —CN Gruppe, oder eine COR Gruppe, in der R für Wasserstoff, einen aliphatischen Rest mit $C_1$—$C_{20}$, aliphatisch-aromatischen Rest mit $C_7$—$C_{20}$, aromatischen Rest mit $C_6$—$C_{20}$ oder heterocyclischen Rest mit 4—16 Ringgliedern und wenigstens einem N, O oder S-Atom steht, bedeuten.

$R^1$ und $R^2$ können beispielsweise Wasserstoff, Fluor, Chlor, Brom, ein Methan-, Äthan-, Hexan-, Cyclohexan-, Propen-, Benzol-, Toluol-, Piperidin-, Morpholin- und Imidazolrest sein und gegebenenfalls zusammen einen Ring mit bis zu acht Gliedern bilden.

A kann vorzugsweise eine —CN-Gruppe, ein Acetyl- oder Benzoyl-, ein Benzol-, Naphthalin- oder Oxazolinrest sein und gegebenenfalls zusammen mit $R^2$ einen bis zu 8 Ringgliedern enthaltenden Zyclus bilden.

Besonders bevorzugt sind $R^1$ und $R^2$ Wasserstoff und A eine Phenylgruppe.

Die erfindungsgemäß für $Y = R^6$—NH ebenfalls als Ausgangsstoffe verwendbaren ungesättigten Carbonsäureamide der allgemeinen Formel (Ib)

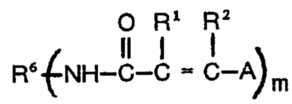

(Ib)

wobei

$R^1$, $R^2$, A die eben angegebene Bedeutung haben, $R^6$ der Definition von $R^5$ entspricht und m für 1 oder 2 steht, können in situ aus den ungesättigten Carbonsäuren (Ia) und organischen Isocyanaten, oder in einer separaten, gegebenenfalls vorgeschaltenen Reaktion in bekannter Weise, beispielsweise aus den ungesättigten Carbonsäuren (Ia) und organischen Isocyanaten, ungesättigten Carbonsäureestern und den Isocyanaten entsprechenden Aminen usw. hergestellt werden.

Als organische Isocyanate können Mono- und/oder Polyisocyanate eingesetzt werden.

Als Monoisocyanate im Sinne der Erfindung werden aliphatische und aromatische, gegebenenfalls durch Heteroatome substituierte Verbindungen mit einer NCO-Gruppe im Molekül eingesetzt, z.B. Alkylisocyanate wie Äthyl-, Methyl-, Butyl-, Dodecyl- und Stearylisocyanat, aromatische, gegebenenfalls substituierte Monoisocyanate wie Phenyl-, Tolyl-, Isopropyl-, Nonylisocyanat, Nitro-, Alkoxy-, Aroxy-, Chlor-, Dichlor-, Trichlor-, Tetrachlor-, Pentachlor-, Benzyl-, Brom-phenyl-isocyanat oder Isocyanatobenzoesäureester, -phthalsäureester, -isophthalsäureester, Isocyanatobenzonitril, cycloaliphatische Isocyanate wie Cyclohexylisocyanat und ungesättigte Isocyanate wie Allyl-, Oleyl-, Cyclohexenyl isocyanat.

Als erfindungsgemäß einzusetzende Ausgangskomponenten kommen ebenso aliphatische, cycloaliphatische, araliphatische, aromatische und heterocyclische Polyisocyanate in Betracht (vgl. Annalen 562, Seiten 75 bis 136).

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Vorzugsweise eignen sich Isocyanate der allgemeinen Formel (V)

$$R^5(\text{—NCO})_n \qquad \text{(V)}$$

in denen $R^5$ für einen, gegebenenfalls mit Halogen, Alkyl- mit $C_1$—$C_{10}$ und/oder Arylgruppen mit $C_6$—$C_{12}$ substituierten Alkylrest mit 2—20 C-Atomen, einen Arylrest mit 5—12 C-Atomen, einen Cycloalkylrest mit 5—12 C-Atomen, einen Alkyl-Arylrest mit 6—20 C-Atomen und einen Heteroatome wie N, O oder S enthaltenden Aryl- oder Cycloalkylrest mit 5—12 C-Atomen steht. n ist eine ganze Zahl von 1—4, vorzugsweise 1—3, besonders bevorzugt 2. Besonders bevorzugt sind aliphatische Rest mit 2—12 C-Atomen oder ein Arylrest wie Phenyl, Tolyl, Naphthyl, Diphenylmethan- und Diphenylätherreste.

Bevorzugt verwendet werden die technisch leicht zugänglichen Gemische aus Toluylendissocyanaten, m-Phenylendiisocyanat, sowie phosgenierte Kondensate aus Anilin und Form-aldehyd mit Polyphenylen-methylen-struktur und die symmetrischen Verbindungen 4,4'-Diisocyanatodiphenylmethan, 4,4'-Diisocyanatodiphenyläther, p-Phenylendiisocyanat und 4,4'-Diisocyanatodiphenyldimethylmethan sowie Isophorondiisocyanat und Hexamethylendiisocyanat.

Die Isocyanate können in freier Form, ferner zum Teil oder vollständig auch in Form ihrer beim Umsatz mit reaktiven Wasserstoff enthaltenden Verbindungen zugänglichen und unter den Reaktionsbedingungen als Isocyanat-Abspalter reagierenden Derivate eingesetzt werden.

Vorzugsweise werden als Abspalter die Additionsprodukte aus Lactamen, Oximen und CH-aciden Verbindungen sowie die aus aliphatischen und aromatischen Mono- und Polyhydroxy-Verbindungen erhaltenen Carbamidsäureester, beispielsweise der allgemeinen Formeln

$$R^5\,(-NH-\underset{\underset{O}{\|}}{C}-O-M)_n \quad \text{bzw.} \quad \left[-\underset{\underset{O}{\|}}{C}-NH-R^5-NH-\underset{\underset{O}{\|}}{C}-O-Q-O-\right]_o$$

eingesetzt, worin $R_5$ und n die oben angegebene Bedeutung haben und M der organische Rest einer Monohydroxyverbindung bzw. Q der organische Rest einer bis- oder trisfunktionellen Hydroxyverbindung, vorzugsweise M und Q, gleich oder verschieden, ein aliphatischer Rest mit 1—10 C-Atomen, ein cycloaliphatischer Rest mit 5—10 C-Atomen, ein aliphatisch-aromatischer Rest mit 7—12 C-Atomen und ein aromatischer Rest mit 6—12 C-Atomen ist, die jeweils noch mit Alkyl- mit $C_1$—$C_{10}$ und/oder Arylgruppen mit $C_6$—$C_{12}$ substituiert sein können, und o für eine ganze Zahl von 1 bis 1000, vorzugsweise von 1 bis 100 steht.

Als Beispiele seien die Carbamidsäureester aus Phenol, isomeren Kresolen, deren technischen Gemischen und ähnlichen aromatischen Hydroxylverbindungen, aliphatische Monoalkohole wie Methanol, Äthanol, Propanol, Isopropanol, Butanol, Isobutanol, Cyclohexanol, Allylalkohol, Benzylalkohol und aliphatische Di- oder Polyole wie Äthylenglykol und Trimethylolpropan, weiterhin die Additionsprodukte mit Pyrrolidon-(2), Caprolactam, Butanon-oxim, Malonester, Acetessigester und Acetophenon aufgeführt.

Die Isocyanat-Abspalter können als solche eingesetzt oder erst in situ durch Umsetzung mit den entsprechenden Reaktanten erzeugt werden.

Anstelle der genannten (Poly)Isocyanate können auch die analogen (Poly)-Isothiocyanate als Ausgangsmaterialien benutzt werden.

Die erfindungsgemäß als Blockierungs- wie auch Lösungsmittel besonders bevorzugten Hydroxylalkyläther sind beispielhaft Verbindungen der allgemeinen Formel (VI)

$$R^8(-OR^9)_q—OH \qquad (VI)$$

in der $R^8$ einen gegebenenfalls substituierten aliphatischen Rest mit $C_1$—$C_{20}$, vorzugsweise $C_1$—$C_8$, cycloaliphatischen Rest mit $C_3$—$C_{10}$, vorzugsweise $C_4$—$C_8$, aliphatisch-aromatischen Rest mit $C_7$—$C_{16}$ oder aromatischen Rest mit $C_6$—$C_{14}$, der z.B. mit Alkoxy-, Aroxy- oder Hydroxy-Gruppen substituiert sein kann, $R^9$ einen aliphatischen Rest mit 2—20 C-Atomen und q eine ganze Zahl von 1—100, bevorzugt 1—4, bedeuten. Vorzugsweise werden erfindungsgemäß solche Hydroxyalkyl-äther eingesetzt, die pro Molekül eine Hydroxy-Gruppe enthalten und in denen $R^9$ einen Rest mit zwei C-Atomen in der Kette, die beispielsweise durch Alkyl-Gruppen substituiert sein können, bedeutet, z.B. die Methyl-, Isopropyl-, Cyclohexyl-, Benzyl-, Phenyl- und Methoxyäthyl-äthylenglykol- und propylenglykol- bzw. diäthylenglykol und -dipropylenglykol-monoäther.

Der erfindungsgemäße Umsatz der ungesättigten Carbonsäuren der Formel (Ia) bzw. Carbonsäureamide der Formel (Ib) mit den organischen Isocyanaten zu den beanspruchten Hydantoinen bzw. Hydantoingruppen enthaltenden Polyiso(thio)cyanaten kann in Lösungsmitteln, die unter den Reaktionsbedingungen nicht reagieren oder lockere, weiterreagierende Additionsverbindungen bilden, oder in einem Überschuß einer der Reaktionskomponente durchgeführt werden.

Außer den bereits aufgeführten Blockierungsmitteln eignen sich als Lösungsmittel Kohlenwasserstoffe, Halogenkohlenwasserstoffe, Phenole, Ester, cyclische Ester, Ketone, Äther, substituierte Amide, Nitrile, beispielsweise Xylole, O-Dichlorbenzol, Phenol, Kresole, Acetophenon, Cyclohexanon, ε-Caprolactam, Äthylenglykolbutyläther, Diäthylenglykolmethyläther, Glykolmonomethylätheracetat, γ-Butyrolacton, ε-Caprolacton, Benzoesäurealkylester, N-Methylpyrrolidon, Dimethylformamid, Dimethylacetamid, Benzonitril und andere sowie deren Gemische. Vorzugsweise werden jedoch keine Lösungsmittel, gegebenenfalls nur stöchiometrische Blockmittelmengen verwendet.

Zur Durchführung der erfindungsgemäßen Verfahren werden die Reaktionskomponenten, gegebenenfalls mit, bevorzugt ohne Lösungsmittel, gegebenenfalls in Gegenwart der Blockierungsmittel, einige Minuten bis zu mehreren Stunden bei Temperaturen von —20°C bis 500°C, bevorzugt 0°C bis 450°C, gehalten. Der Reaktionsverlauf kann über die Gasentwicklung und die IR-Spektren verfolgt werden.

Die hohe Geschwindigkeit der erfindungsgemäßen Umsetzung gestattet auch deren Durchführung in Gegenwart phenolischer und/oder alkoholischer Hydroxylgruppen und/oder lactonischer Amidgruppen, d.h. derartige Gruppierungen können in den Umsetzungskomponenten, gegebenenfalls als Blockmittel vorhanden sein, bzw. überschüssige Hydroxyl- bzw. Lactam-Komponenten auch als Lösungsmittel verwendet werden.

Die Acidität saurer Lösungsmittel wie der Phenole bzw. Kresole genügt zur Reaktionsführung innerhalb hinreichend kurzer Reaktionszeiten. In inerten Medien oder in Schmelzansätzen können beispielsweise Carbonsäuren mit hinreichendem Schmelz- bzw. Siedepunkt wie Essigsäure, Benzolsäure, Bernsteinsäure, Benzoldicarbonsäuren, Butantetracarbonsäure, Trimelitsäure bzw. deren Anhydride gegebenenfalls auch als einbaufähige Katalysatoren in Mengen von 0,1 bis 40, vorzugsweise von 1 bis 10 Prozent in Val, bezogen auf ein Val Isocyanat, mitverwendet werden.

Zur weiteren Beschleunigung der ablaufenden Reaktionen sind die aus der Isocyanatchemie geeigneten bekannten Basen, Säuren und/oder Metallkatalysatoren wie Triäthylamin, N-Methylmorpholin, Endoäthylenpiperazin, Titantetrabutylat, Titanaminoalkohol, Eisenacetylacetonat, Dibutylzinnlaurat oder p-Tolyolsulfonsäure zu verwenden.

Zuweilen ist es vorteilhaft, die Reaktionen in mehreren Stufen durchzuführen oder die einzelnen Komponenten in unterschiedlicher Reihenfolge, gegebenenfalls bei verschiedenen Temperaturen, zuzusetzen. So kann beispielsweise in einer ersten Stufe, gegebenenfalls in einem Lösungs- und/oder Blockmittel ein Addukt oder Kondensat hergestellt werden, das dann bei höherer Temperatur, eventuell unter Verdampfung des Lösungsmittels, gegebenenfalls nach Zusatz latenter Blockierungsmittel unter Cyclisierung und/oder Kettenverlängerung und/oder Vernetzung in das gegebenenfalls hochmolekulare Kondensationsprodukt übergeht. Wird dieses dann zur Beschichtung verwendet, so kann es auch aus Schmelzen oder wäßrigen Systemen appliziert werden.

Mitunter wird die Umsetzung zweckmäßigerweise unter einem inerten Schutzgas wie $N_2$ oder Argon durchgeführt.

Schließlich kann die erfindungsgemäße Reaktion in kontinierlicher oder diskontinuierlicher Ausführung oder gegebenenfalls in Autoklaven unter Druck zum Zwecke einer höheren Reaktionstemperatur erfolgen.

Im allgemeinen wird entsprechend der erfindungsgemäßen Reaktion, vorteilhafterweise pro funktioneller —COOH— sowie intermediar entstehender oder gegebenenfalls separat erzeugter —CO—NH-Gruppe mindestens ein Äquivalent Isocyanat eingesetzt.

Es gehört jedoch mit zur Ausführungsform der vorliegenden Verfahren, das NCO—/funktionelle —COOH— und/oder —CONH-Gruppen-verhältnis auch innerhalb der Molverhältnisse von $\geqq$ 2:1 bis 1:1 vorzugeben. Um das Eigenschaftsbild der erfindungsgemäßen Polyisocyanate jedem möglichen Anwendungsfall anpassen zu können, sind jedoch auch sehr weitgehende Abweichungen von diesem Molverhältnis möglich. Allerdings sind nur solche Molverhältnisse zweckmäßig, die zumindest schmelzbare und/oder in organischen Lösungsmitteln universell lösliche Produkte ergeben.

Ferner beansprucht eine weitere Ausführungsform der vorliegenden Verfahren die Verwendung beliebiger Isocyanatgemische, so daß beispielsweise auch zur Amid- sowie zur Hydantoinringbildung jeweils andere organische Isocyanate eingesetzt werden können.

Die ungesättigten Carbonsäuren der Formel (Ia), bzw. entsprechende Carbonsäureamide der Formel (Ib) liefern mit monofunktionellen Isocyanaten monomolekulare Hydantoine, mit Diisocyanaten in Abhängigkeit von den stöchiometrischen Verhältnissen höher- und hochmolekulare, z.T. vernetzte Polyhydantoine, gegebenenfalls auch oligo- und monomere Hydantoingruppen enthaltende Polyisocyanate.

Im Rahmen des erfindungsgemäßen Verfahrens können Produkte mit gegebenenfalls verkappten Isocyanatgruppen entstehen.

Darüber hinaus ist es auch möglich, Produkte mit vergleichbarem Polymerisationsgrad, aber geringerem, gegebenenfalls verkapptem Isocyanatgehalt, durch Mitverwendung berechneter Anteile an Monoisocyanaten herzustellen. Geeignet sind beispielsweise Phenylisocyanat, $\alpha$-Naphthylisocyanat, Isocyanatobenzoesäureester, Isocyanatoessigsäureester usw.

Eine Aufarbeitung der z.T. relativ hochmolekularen, gegebenenfalls mit Isocyanat- bzw. Carbamidsäurederivaten sowie Carbonsäureester, -amid- oder -amidurethanresten substituierten bzw. modifizierten Hydantoinen ist im Prinzip in der üblichen Weise, etwa durch Kristallisationsverfahren möglich, meist aber nicht erforderlich, da diese Produkte, gegebenenfalls auskondensiert, gegebenenfalls in statu nascendi, gegebenenfalls nach vorheriger Umwandlung ihrer Säure- und/oder Alkoholfunktionen, gegebenenfalls erst durch Kombination mit gegebenenfalls mehrwertigen funktionellen Aminen, gegebenenfalls mehrwertigen Alkoholen wie beispielsweise Äthylenglykol, Trimethylolpropan, Glycerin, Trishydroxyäthylisocyanurat und/oder gegebenenfalls mehrwertigen Carbonsäuren bzw. deren Anhydriden wie beispielsweise O-Phthalsäure, Isophthalsäure, Terephthalsäure, Trimelitsäure, Butantetracarbonsäure, gegebenenfalls unter Einsatz weiterer Polyisocyanate bzw. Polyisocyanat-Abspalter, in der üblichen Weise zu linearen und/oder verzweigten Kunststoffen mit universeller Löslichkeit, höherer Temperaturbeständigkeit, guter Elastizität und gutem Hitzeschockverhalten umgesetzt werden können.

Mit analogem Erfolg können die erfindungsgemäßen Kondensationsprodukte, gegebenenfalls auch ihre Vorstufen, Polyestern aus O-Phthalsäure/Terephthalsäure/Isophthalsäure, bzw. deren Ester,

Äthylenglykol, Glycerin/Trimethylolpropan/Trishydroxyäthylisocyanurat, Polyäther aus Äthylenoxid und/oder Bis-(hydroxyphenyl)-propan und Epichlorhydrin, Polyurethanen, Polyamiden usw. zugemischt und gegebenenfalls an- und/oder einkondensiert werden. In allen Fällen entstehen dabei modifizierte Polymere, die neben den (Thio)-Hydantoinringen gegegebenenfalls zusätzliche Äther-, Carbamidsäurederivat-, Carbonsäureester-, -amid-, -imid-, -esteramid-, -esterimid-, -amidimid- und/oder -esteramidimidgruppierungen enthalten.

Die Mengenverhältnisse dieser Zusätze können in weiten Bereichen schwanken, vorzugsweise werden bezüglich des erfindungsgemäßen Kondensates 10—400 Gew.-% eingesetzt.

Die nach der erfindungsgemäßen Verfahrenstechnik zugänglichen nieder- bzw. monomolekularen Hydantoine besitzen biochemische Wirkungen, die erfindungsgemäßen Polyhydantoine eine besondere Temperaturbeständigkeit.

Die mit den erfindungsgemäßen Polykondensaten modifizierten Kunststoffe zeigen verbesserte Temperaturverhalten sowie universellere Löslichkeit, unter anderem auch in umweltneutralen Lösungsmitteln. Die Polymeren können zur Herstellung temperaturbeständiger Kleber, Lacke, Folien, Pulver, und Kunststoffe sowie zur Beschichtung hitzeresistenter Substrate verwendet werden. Ihr Eigenschaftsprofil kann je nach Einsatzgebiet durch Zusatz von Füllstoffen, Pigmenten, nieder- und hochmolekularen Komponenten in weiten Grenzen variiert werden.

## Beispiel 1

307,1 g 4-Chlorphenylisocyanat werden unter $N_2$ zunächst bei Raumtemperatur mit

410 g $\gamma$-Butyrolacton und

4,6 g o-Dichlorbenzol, dann ab 10—30°C im Zuge der $CO_2$-Entwicklung mit

148,2 g Zimtsäure vermischt, 1 Stunde bei 50°C nachgerührt, mit

0,5 g Endoäthylenpiperazin versetzt und dann über 70/100/120/150/175°C, schließlich auch unter Rückfluß bis zum Ende der $CO_2$-Entwicklung sowie zum völligen Verbrauch der NCO-Gruppe gerührt.

Zur Aufarbeitung destilliert man die Lösungsmittel i.Vakuum ab und kristallisiert den verbliebenen Rückstand aus Äthanol/Petroläther um.

Es resultieren nahezu farblose bis schwach gelbe Kristalle vom FP: 178—80°C, deren IR-Spektrum die für Hydantoine typischen Banden bei 1710 und 1755 $cm^{-1}$ aufweisen. Die auf 1,3-Bis-(4-Chlorphenyl-)5-benzylhydantoin berechnete Analyse erfolgt für $C_{22}H_{16}N_2O_2Cl_2$ (411,3):

| | | | | |
|---|---|---|---|---|
| ber.: | C 64,2 | H 3,9 | N 6,8 | Cl 17,2 |
| gef.: | 64,5 | 4,0 | 6,6 | 16,7 |

## Beispiel 2

169 g Mucochlorsäure werden unter $N_2$ zunächst bei 25°C in

500 g $\gamma$-Butyrolacton gelöst, dann bei 30 bis $\leq$ 50°C mit der Lösung von

250,2 g 4,4'-Diisocyanatodiphenylmethan in

200 g Toluol versetzt.

Man rührt bei 30°C nach, heizt dann im Zuge der Gas-Entwicklung schließlich solange bei 170—190°C, bis der NCO-Test negativ verläuft.
Mit steigender Viskosität wird zunächst mit

X g $\gamma$-Butyrolacton, abschließend dann bei 140—120°C mit insgesamt

(375—X)g $\gamma$-Butyrolacton verdünnt und noch ca. 1 h bei 200°C nachgerührt.

Die Viskosität der resultierenden Lösung beträgt 1730 $cP_{20°C}$. Ein mit dieser Lacklösung beschichtetes und bei 250°C während 15 Minuten und bei 300°C während 10 Minuten eingebranntes Tiefziehblech besitzt einen elastischen, gut haftenden Lackfilm von guter Oberflächenhärte und ausgezeichneter Lösungsmittelfestigkeit.

Beispiel 3

296,3 g Zimtsäure werden unter $N_2$ in

500 g γ-Butyrolacton gelöst, dann bei 30 bis ≦50°C mit

222,0 g Isophorondiisocyanat versetzt und solange bei 150 bis 180°C gerührt, bis der NCO-Test negativ verläuft. Anschließend versetzt man bei ca. 70°C mit

250,2 g 4,4'-Diisocyanatodiphenylmethan und

0,5 g Triäthylendiamin und heizt schließlich ca. 9 Stunden bei 200—205°C bzw. bis zum Verbrauch aller NCO-Gruppen.

Im Zuge steigender Viskosität kann mit zunächst

X g Acetophenon, abschließend dann bei 170—150°C mit

(1588—X)g Benzoesäuremethylester verdünnt werden. Die nochmals 1 Stunde bei 170°C nachgerührte Lacklösung besitzt eine Viskosität von ca. 1270 $cP_{20°C}$.

Das nach der Methanolfällung verbleibende Bindemittel besitzt im IR-Spektrum die den Hydantoinen eigenen Banden.

Der aus obiger Lacklösung in einem 4 m Ofen bei 9 m/Minute hergestellte 0,7 mm Cu-Lackdraht hat eine Erweichungstemperatur von > 330°C, einen Hitzeschock von > 220°C und eine Dauerwärmebeständigkeit von mindestens 14 Tagen 180°C.

**Patentansprüche**

1. Verfahren zur Herstellung von Verbindungen, die aus 1—1000 mal wiederkehrenden Struktureinheiten der allg. Formel IIa und IIb

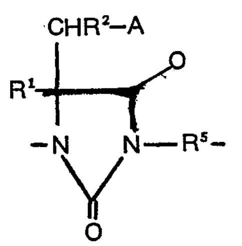 (IIa) 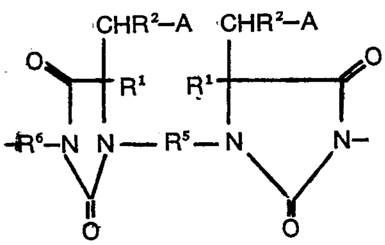 (IIb)

bestehen, die ggf. über wenigstens einen $R^5$- oder $R^6$-Rest verknüpft sind und ggf. endständige NCO-Gruppen aufweisen, wobei die Reste

$R^1$ und $R^2$ gleich oder verschieden, Wasserstoff, Halogen, einen gegebenenfalls substituierten aliphatischen, aliphatisch-aromatischen, aromatischen oder heterocyclischen Rest

A eine —CN-Gruppe, eine —COR-Gruppe- mit R = Wasserstoff, ein aliphatischer, aliphatisch-aromatischer, aromatischer oder heterocyclischer Rest; oder einen gegebenenfalls substituiertenaromatischen, aliphatischen, aliphatisch-aromatischen oder heterocyclischen Rest bedeutet, und

$R^5$ und $R^6$ für einen gegebenenfalls substituierten aliphatischen, aliphatisch-aromatischen, aromatischen oder heterocyclischen Rest stehen, dadurch gekennzeichnet, daß organische Isocyanate der allg. Formel (V)

$$R^5(-NCO)_n \qquad (V)$$

bzw. Isocyanat-Abspaltern mit ungesättigten Carbonsäuren bzw. -Derivaten der allgemeinen Formeln

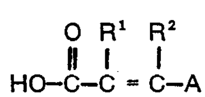 (Ia) oder (Ib) 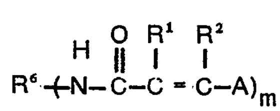 ,

worin

$R^1$, $R^2$, A, $R^5$ und $R^6$ die oben angegebene Bedeutung haben,
m 1 oder 2 und
n eine ganze Zahl von 1—4 bedeutet,

gegebenenfalls in Anwesenheit eines Katalysators und ein oder mehreren Lösungsmitteln bei Temperatur von —20 bis 500°C umgesetzt werden.

2. Verfahren nach Anspruch 1, dadurch gekennzeichnet, daß in den allgemeinen Formeln $R^1$ und $R^2$ gleich oder verschieden, Wasserstoff, Halogen, einen aliphatischen Rest mit $C_1$—$C_{20}$, aliphatisch-aromatischen Rest mit $C_7$—$C_{20}$, aromatischen Rest mit $C_6$—$C_{20}$ oder heterocyclischen Rest mit 4 bis 16 Ringgliedern und wenigstens einem N, O oder S-Atom und

A einen aliphatischen Rest mit $C_1$—$C_{20}$, einen aliphatisch-aromatischen Rest mit $C_7$—$C_{20}$, einen aromatischen Rest mit $C_6$—$C_{20}$, der heterocyclischen Rest mit 4—16 Ringgliedern, die gegebenenfalls durch Halogen, —$NO_2$, —$COOR_4$, —CN, —CO, —OH Gruppen mit $R_4 = R_1$ außer Halogen, substituiert sein können; eine —CN Gruppe, oder eine COR Gruppe, in der R für Wasserstoff, einen aliphatischen Rest mit $C_1$—$C_{20}$, aliphatisch-aromatischen Rest mit $C_7$—$C_{20}$, aromatischen Rest mit $C_6$—$C_{20}$ oder heterocyclischen Rest mit 4—16 Ringgliedern und wenigstens einem N, O oder S-Atom, und

$R^6$ einen aliphatischen Rest mit 2—20 C-Atomen, einen aromatischen Rest mit 5—12 C-Atomen, einen cycloaliphatischen Rest mit 5—12 C-Atomen, einen aliphatisch-aromatischen Rest mit 6—20 C-Atomen und einen Heteroatome wie N, O oder S enthaltenden aromatischen oder cycloaliphatischen Rest mit 5—12 C-Atomen, die alle gegebenenfalls mit Halogen, Alkyl- mit $C_1$—$C_{10}$ und/oder Arylgruppen mit $C_6$—$C_{12}$ substituiert sein können, bedeuten.

3. Verfahren nach Ansprüchen 1 und 2, dadurch gekennzeichnet, daß als ungesättigte Säure der allg. Formel Ia Zimtsäure eingesetzt wird.

4. Hydantoine erhältlich nach Verfahren nach Ansprüchen 1—3.

5. Hydantoinpolyisocyanate erhältlich nach Verfahren gemäß Ansprüchen 1—3.

**Revendications**

1. Procédé pour la préparation de composés qui consistent en motifs récurrents répétés 1—1000 fois de formules

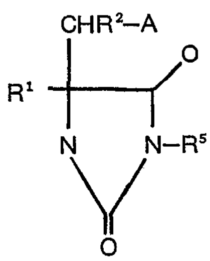

(IIa)

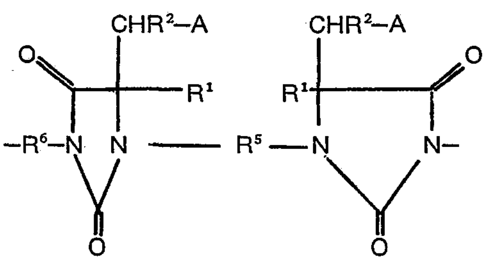

(IIb)

qui sont liés éventuellement par l'intermédiaire d'au moins un reste $R^5$ ou $R^6$ et présentent des groupes NCO terminaux,

dans lesquelles

$R^1$ et $R^2$ sont identiques ou différents et représentent l'hydrogène, un halogène, un reste aliphatique, aliphatico-aromatique, aromatique ou hétérocyclique éventuellement substitué,

A représente un groupe —CN, un groupe —COR où R = l'hydrogène, un reste aliphatique, aliphatico-aromatique, aromatique ou hétérocyclique, ou un reste aromatique, aliphatique, aliphatico-aromatique, ou hétérocyclique éventuellement substitué, et

$R^5$ et $R^6$ représentent un reste aliphatique, aliphatico-aromatique, aromatique ou hétérocyclique éventuellement substitué,

caractérisé en ce que l'on fait réagir des isocyanates organiques de formule générale

$$R^5(\text{—NCO})_n \qquad (V)$$

ou des générateurs d'isocyanates avec des acides carboxyliques insaturés ou des dérivés de formules générales

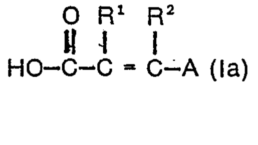 (Ia) ou (Ib) 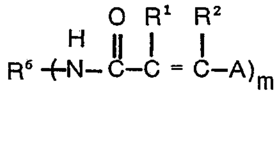

dans lesquelles

$R^1$, $R^2$, A, $R^5$ et $R^6$ ont la signification indiquée ci-dessus,

m est égal à 1 ou 2 et

n est un nombre entier de 1—4,

éventuellement en présence d'un catalyseur et d'un ou plusieurs solvants, à une température de −20 à 500°C.

2. Procédé selon la revendication 1, caractérisé en ce que, dans les formules générales,

$R^1$ et $R^2$ sont identiques ou différents et représentent l'hydrogène, un halogène, un reste aliphatique, en $C_1$—$C_{20}$, un reste aliphatico-aromatique en $C_7$—$C_{20}$, un reste aromatique en $C_6$—$C_{20}$ ou un reste hétérocyclique à 4—16 chaînons et au moins un atome N, O ou S, et

A représente un reste aliphatique en $C_1$—$C_{20}$, un reste aliphatico-aromatique en $C_7$—$C_{20}$, un reste aromatique en $C_6$—$C_{20}$ ou un reste hétérocyclique à 4—16 chaînons, qui peuvent éventuellement être substitués par un halogène, des groupes $—NO_2$, $—COOR_4$, —CN, —CO, —OH avec $R_4 = R_1$ sauf un halogène, un groupe —CN ou un groupe COR, dans lequel R représente l'hydrogène, un reste aliphatique en $C_1$—$C_{20}$, un reste aliphatico-aromatique en $C_7$—$C_{20}$, un reste aromatique en $C_6$—$C_{20}$ ou un reste hétérocyclique à 4—16 chaînons et au moins un atome N, O ou S, et

$R^6$ représente un reste aliphatique en $C_2$—$C_{20}$, un reste aromatique en $C_5$—$C_{12}$, un reste cycloaliphatique en $C_5$—$C_{12}$, un reste aliphatico-aromatique en $C_6$—$C_{20}$ et un reste aromatique ou cycloaliphatique en $C_5$—$C_{12}$ contenant des hétéroatomes tels que N, O ou S, qui peuvent tous être éventuellement substitués par un halogène, des groupes alkyle en $C_1$—$C_{10}$ et/ou des groupes aryle en $C_6$—$C_{12}$.

3. Procédé selon les revendications 1 et 2, caractérisé en ce que l'on utilise comme acide insaturé de formule générale la l'acide cinnamique.

4. Hydantoïnes pouvant être obtenues par des procédés selon les revendications 1—3.

5. Hydantoïne-polyisocyanates pouvant être obtenus par des procédés selon les revendications 1—3.

**Claims**

1. Process for the preparation of compounds consisting of structural units of the general formula IIa and IIb

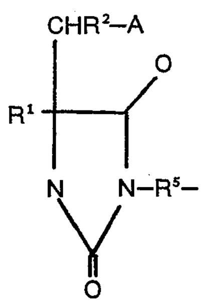

(IIa)

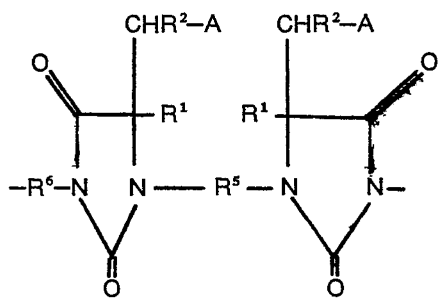

(IIb)

which recur once to 1,000 times and are optionally linked via at least one $R^5$ or $R^6$ radical and optionally contain terminal NCO groups,

in which

the radicals $R^1$ and $R^2$ are identical or different and denote hydrogen, halogen or an optionally substituted aliphatic, aliphatic-aromatic, aromatic or heterocyclic radical,

A denotes a —CN group, a —COR group, with R = hydrogen, or an aliphatic, aliphatic-aromatic, aromatic or heterocyclic radical; or an optionally substituted aromatic, aliphatic, aliphatic-aromatic or heterocyclic radical, and

$R^5$ and $R^6$ represents an optionally substituted aliphatic, aliphatic-aromatic, aromatic or heterocyclic radical,

characterised in that organic isocyanates of the general formula (V)

$$R^5(—NCO)_n \quad (V)$$

or agents which split off isocyanate, are reacted with unsaturated carboxylic acids or unsaturated carboxylic acid derivatives of the general formulae

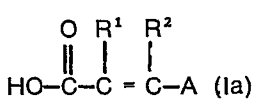

(Ia) or (Ib)

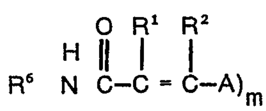

wherein

$R^1$, $R^2$, A, $R^5$ and $R^6$ have the abovementioned meaning,

m denotes 1 or 2 and

n denotes an integer from 1 to 4,

if appropriate in the presence of a catalyst and one or more solvents at temperatures from −20 to 500°C.

2. Process according to Claim 1, characterised in that, in the general formulae,

$R^1$ and $R^2$ are identical or different and denote hydrogen, halogen, a $C_1$—$C_{20}$-aliphatic radical, a $C_7$—$C_{20}$-aliphatic-aromatic radical, a $C_6$—$C_{20}$-aromatic radical or a heterocyclic radical with 4 to 16 ring members and at least one N, O or S atom, and

A denotes a $C_1$—$C_{20}$-aliphatic radical, a $C_7$—$C_{20}$-aliphatic-aromatic radical, a $C_6$—$C_{20}$-aromatic radical or a heterocyclic radical which has 4 to 16 ring members, it being possible for these radicals optionally to be substituted by halogen, —$NO_2$, —$COOR_4$, —CN, —CO or —OH groups, with $R_4 = R_1$, with the exception of halogen; or a —CN group, or a COR group,

in which

R represents hydrogen, a $C_1$—$C_{20}$-aliphatic radical, a $C_7$—$C_{20}$-aliphatic-aromatic radical, a $C_6$—$C_{20}$-aromatic radical or a heterocyclic radical with 4—16 ring members and at least one N, O or S atom, and

$R^6$ denotes an aliphatic radical with 2—20 C atoms, an aromatic radical with 5—12 C atoms, a cycloaliphatic radical with 5—12 C atoms, an aliphatic-aromatic radical with 6—20 C atoms, or an aromatic or cycloaliphatic radical which has 5—12 C atoms and contains a hetero-atom, such as N, O or S, it being possible for all these radicals optionally to be substituted by halogen, $C_1$—$C_{10}$-alkyl groups and/or $C_6$—$C_{12}$-aryl groups.

3. Process according to Claims 1 and 2, characterised in that cinnamic acid is employed as the unsaturated acid of the general formula Ia.

4. Hydantoins which can be obtained by the process according to Claims 1—3.

5. Hydantoin polyisocyanates which can be obtained by the process according to Claims 1—3.